## Europäisches Patentamt

## European Patent Office

## Office européen des brevets

(11) Veröffentlichungsnummer: **0 108 962**
**A2**

(12) **EUROPÄISCHE PATENTANMELDUNG**

(21) Anmeldenummer: 83110494.8

(22) Anmeldetag: 21.10.83

(51) Int. Cl.³: **G 01 N 25/08,** G 01 N 27/18, G 01 N 33/28, B 60 T 17/22 // G01K7/18, G01K11/02

(30) Priorität: 06.11.82 DE 3241078

(43) Veröffentlichungstag der Anmeldung: 23.05.84 Patentblatt 84/21

(84) Benannte Vertragsstaaten: **AT BE CH DE FR GB IT LI NL SE**

(71) Anmelder: **Battelle-Institut e.V., Am Römerhof 35 Postfach 900 160, D-6000 Frankfurt/Main 90 (DE)**

(72) Erfinder: **Hartmann, Rolf, Am Hohenstein 2, D-6233 Kelkheim (DE)**

(74) Vertreter: **Rupprecht, Klaus, Dipl.-Ing., Am Römerhof 35, D-6000 Frankfurt (Main) 90 (DE)**

(54) Verfahren und Anordnung zur Überwachung von Bremsflüssigkeiten im Hinblick auf deren Siedepunktserniedrigung.

(57) Bei einem Verfahren zur Überwachung von Bremsflüssigkeiten im Hinblick auf deren Siedepunkterniedrigung durch Wasseraufnahme, wird in die Bremsflüssigkeit eine elektrisch betriebene Heizstrecke mit vergleichsweise geringen Abmessungen eingebracht. Die die Heizstrecke unmittelbar umgebende Flüssigkeitsmenge wird bis zum örtlichen Sieden erhitzt, wobei die Temperatur der Heizstrecke durch das örtliche Sieden auf einen konstanten Wert festgehalten wird. Der elektrische Widerstand der Heizstrecke bei dieser konstanten Temperatur wird als ein Maß für den Siedepunkt der Bremsflüssigkeit benutzt und die Unterschreitung eines Sollwerts des Siedepunkts angezeigt.

- 1 -

392-93                                    18. August 1982
CASCH/AGD/UMA

BATTELLE - INSTITUT E.V., Frankfurt/Main


=========================================
Verfahren und Anordnung zur Überwachung
von Bremsflüssigkeiten im Hinblick
auf deren Siedepunkterniedrigung
=========================================


Die Erfindung betrifft ein Verfahren und eine Anordnung zur Überwachung von Bremsflüssigkeiten im Hinblick auf deren Siedepunkterniedrigung durch Wasseraufnahme.

Der minimal zulässige Siedepunkt von Bremsflüssigkeiten darf in keinem der hitzegefährdeten Teile, z.B. in Radbremszylindern von Kraftfahrzeugen, unterschritten werden, da dann die Gefahr besteht, daß bei starker Bremsbelastung die Flüssigkeit zu sieden beginnt und somit die Bremswirkung durch Bildung von Dampfblasen vollständig ausfällt.

Die Bremsflüssigkeiten von Kraftfahrzeugen werden zur Zeit durch Probennahme am Vorratsbehälter und externe Siedepunktbestimmung kontrolliert. Der Inhalt des Vorratsbehälters muß aber nicht notwendigerweise die gleiche Qualität wie der der Radbremszylinder haben, da die Bremsflüssigkeit weder umgewälzt noch durchmischt wird. Reichert sich z.B. wegen einer defekten Manschette der Inhalt eines Radbremszylinders mit 3-5 % Wasser an, so kann bei starker Bremsbelastung dessen Inhalt zum Sieden kommen und die Bremsanlage außer Funktion setzen, während im Vorratsbehälter die Bremsflüssigkeit noch keine Siedepunkterniedrigung zeigt.

Der vorliegenden Erfindung liegt nun die Aufgabe zugrunde, ein Verfahren und eine Anordnung zu entwickeln, mit denen eine Kontrolle der Bremsflüssigkeit zuverlässig und ohne großen Aufwand durchführbar ist.

Diese Aufgabe ist erfindungsgemäß dadurch gelöst, daß in die Bremsflüssigkeit eine elektrisch betriebene Heizstrecke mit vergleichsweise geringen Abmessungen eingebracht und die die Heizstrecke unmittelbar umgebende Flüssigkeitsmenge bis zum örtlichen Sieden erhitzt wird, wobei die Temperatur der Heizstrecke durch das örtliche Sieden auf einen konstanten Wert festgehalten wird und daß der elektrische Widerstand der Heizstrecke bei dieser konstanten Temperatur als ein Maß für den Siedepunkt der Bremsflüssigkeit benutzt und die Unterschreitung eines Sollwerts des Siedepunkts angezeigt wird. Vorteilhafte Weiterbildungen des erfinderischen Verfahrens werden in den Unteransprüchen 2 bis 4 erläutert.

In der erfindungsgemäßen Anordnung zur Durchführung dieses Verfahrens sind innerhalb eines, die zu überwachende Bremsflüssigkeit enthaltenden Behälters eine direkt oder indirekt elektrisch betriebene und von der Bremsflüssigkeit allseitig umgebene Heizstrecke mit vergleichsweise geringen Abmessungen, deren elektrische Anschlüsse druckdicht und elektrisch isoliert durch die Behälterwandung geführt sind, ferner außerhalb eine Elektronik zur Stromversorgung und zur Bestimmung des Widerstands der Heizstrecke sowie eine Meßwertanzeige vorgesehen.

Vorteilhafte Ausbildungen der erfindungsgemäßen Anordnung sind in den Unteransprüchen 6 bis 8 beschrieben.

Das erfindungsgemäße Meßprinzip beruht darauf, daß durch Erwärmung einer sehr kleinen Flüssigkeitsmenge der aktuelle Wert des Siedepunkts über eine Widerstandsmessung der Heizstrecke bestimmt wird. Die erfindungsgemäße Anordnung wird bei Kraftfahrzeugen vorzugsweise in jeden der Radbremszylinder eingebaut und ermöglicht daher eine Überwachung der Bremsflüssigkeit an Ort und Stelle.

Gemäß einer Ausführungsform besteht die erfindungsgemäße Anordnung aus einer druckdicht und elektrisch isoliert eingebauten Wendel eines dünnen Metalldrahts, z.B. Nickel, Edelstahl oder dgl. der allseitig von der Bremsflüssigkeit umspült wird. Die Dimension des Drahts im Vergleich zu dem Volumen der Bremsflüssigkeit muß sehr gering sein. Dieser Draht wird von einem elektrischen Strom durchflossen, der hoch genug gewählt wird, um unmittelbar an der Oberfläche des Drahts die Flüssigkeit so weit zu erwärmen, daß örtliches Sieden einsetzt. Als örtliches bzw. partielles Sieden wird erfindungsgemäß der folgende Vorgang verstanden: Wenn eine Flüssigkeit durch eine untergetauchte Heizstrecke allmählich erwärmt wird, beobachtet man, daß zu einem bestimmten Zeitpunkt Dampfbläschen an der Heizfläche erscheinen. Diese verschwinden aber wieder, nachdem sie eine kleine Strecke in der Flüssigkeit hochgestiegen sind. Offenbar hat die Flüssigkeit in einiger Entfernung von der Heizfläche die Verdampfungstemperatur noch nicht erreicht, und der Dampf in den Bläschen kondensiert wieder, nachdem sich diese von der Heizstrecke getrennt haben. Durch das örtliche Sieden wird dem Draht soviel Energie entzogen, daß sich die Temperatur auf die Siedetemperatur der Flüssigkeit stabilisiert. Da andererseits der elektrische Widerstand des Drahts mit einem Temperaturkoeffizienten behaftet ist, wird auch der aktuelle Widerstand des Drahtes, der im Falle von Nickel bei Stromdurchgang zunächst ansteigt, auf einem bestimmten, der Siedetemperatur zuzuordnenden Widerstand verharren.

- 4 -

Dieser Effekt ist besonders deutlich, wenn an Stelle eines konstanten Stromes ein kontinuierlich ansteigender Strom benutzt wird, wobei eine geeignete elektronische Schaltung den Drahtwiderstand im Moment des Verharrens bestimmt. Als Indikator für den Effekt des einsetzenden örtlichen Siedens kann auch die Fluktuationen des Widerstands dienen, die durch die Bildung und den Zerfall von Dampfblasen über dadurch bedingte Temperaturschwankungen des Drahts verursacht werden. Ist nun die Bremsflüssigkeit mit Wasser verunreinigt, so wird sie früher zu sieden beginnen und der als Siedepunkt-Sensor dienende Heizdraht wird eine niedrigere Temperatur melden, die dann gegebenenfalls in Form eines Warnsignals den Fahrer zum Austausch der verdorbenen Flüssigkeit auffordert.

Trotz der Blasenbildung bei der Bestimmung des Siedepunktes gefährdet dieses System auch dann nicht die Verkehrssicherheit des Fahrzeuges, wenn während der kurzfristigen Messung die Bremse bedient wird. Die hier erzeugten Dampfblasen sind mikroskopisch klein, die zugeführte Leistung im Vergleich zur Bremsleistung verschwindend gering. Die durch den Druckanstieg beim Bremsen allerdings durch die Siedepunkterhöhung bewirkte Fehlmessung kann ignoriert werden, wenn die Meßelektronik so ausgelegt ist, daß eine Messung z.B. in periodischen zeitlichen Abständen und nur bei dem Signal "Bremse gelöst" (Bremslichtschalter offen) durchgeführt wird. Die Messung selbst wird in höchstens einigen Sekunden durchgeführt.

Ist Material und Temperaturkoeffizient des Sensordrahts bei der Herstellung bekannt und wird der Sensor dann auf einen festen Nullwert bei Raumtemperatur abgeglichen, so ist später keinerlei Abgleich oder Kompensation z.B. der Umgebungstemperatur erforderlich; jeder Widerstand des Drahts ist eindeutig einer Temperatur, d.h. einem Bremsflüssigkeits-Siedepunkt zuzuordnen.

Bei Verwendung von Nickel als Sensormaterial mit einem Temperaturkoeffizienten von $6,7 \times 10^{-3} \ K^{-1}$ kann bereits ein sehr deutliches Signal in Form einer Widerstandserniedrigung bei Verunreinigung der Bremsflüssigkeit durch Wasser erhalten werden. Wird an Stelle des Metalls z.B. ein Halbleitermaterial, ein Kaltleiter mit steilem Widerstandsanstieg im interessanten Temperaturbereich verwendet, so kann die Nachweisempfindlichkeit in Form einer Schwelle "gut/schlecht" ohne großen Aufwand noch erhöht werden.

Wird eine für Bremsflüssigkeiten noch zulässige Mindest-Siedetemperatur als Warnschwelle eingestellt, so kann dieses System dann mit allen Arten von Bremsflüssigkeiten ohne Einschränkung arbeiten und zuverlässig bei Qualitätsverlust durch Wasseraufnahme ein Alarmsignal auslösen.

Die Erfindung wird anhand beiliegender Zeichnung, die ein Ausführungsbeispiel zeigt, näher erläutert.

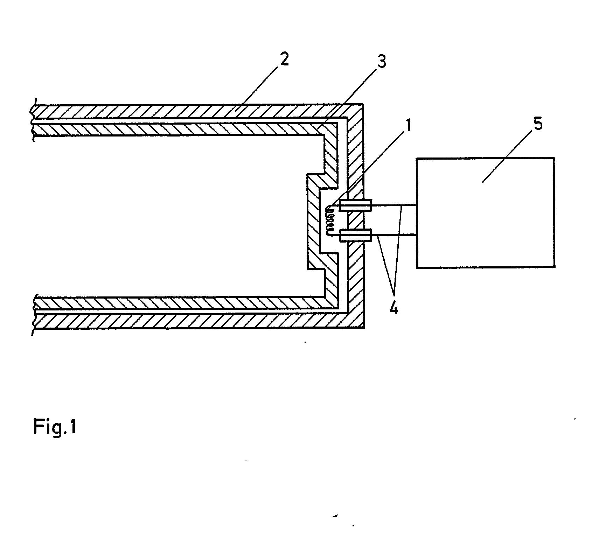

In Fig. 1 wird als Heizstrecke 1 in einen Radbremszylinder 2 eingebaut. Der korrespondierende Teil des Kolbens 3 wird vorzugsweise mit einer kleinen und den Abmessungen der Heizstrecke 1 entsprechenden Einbuchtung versehen. Die elektrischen Leitungen 4 des Sensors führen zur Elektronik 5. Wesentliche Teile der Elektronik bestehen an der Stromsteuerung und dem Grenzwertgeber sowie der Warnung, z.B. in Form eines am Armaturenbrett angebrachten Kontrollämpchens. Ein Wahlschalter ermöglicht die Messung in verschiedenen Zylindern, in denen ebenfalls Sensoren vorgesehen sind. Eine Programmsteuerung dient zur Messung in vorgegebenen Zeit- oder Kilometerabständen.

Da es sinnvoll sein kann, die Messung in einem Bremszylinder mehrere Male zu wiederholen, wird eine Wiederholschaltung angebracht. Eine Logik steuert, daß die Messung nur dann durchgeführt wird, wenn keine Bremswirkung vorliegt.

Zur Kontrolle einer konventionellen Bremsflüssigkeit (z.B.DOT 3, DOT 4, DOT 5) beträgt der Prüfbereich der Meßschaltung z.B. 150 bis 200°C. Wenn die Bremsflüssigkeit gut ist, d.h. Siedepunkt über 200°C liegt, kann ein Überhitzen über 200°C vermieden werden, um die Flüssigkeit zu schonen. Liegt der Siedepunkt z.B. um 170°C oder tiefer, wird bei unterschreiten eines Grenzwertes der Fahrer informiert.

Die Heizstrecke besteht in diesem Beispiel aus einem gewendelten Nickel-Draht. Gesamtwendellänge beträgt ca. 3 mm bei ca. 1 mm Wendeldurchmesser. Die Abmessungen variieren in Abhängigkit von der Stromversorgung. In diesem Beispiel wird von z.B. 0 Watt steigend so viel Strom zugeführt, bis örtliches Sieden einsetzt. Der gemessene Spannungsabfall ist direkt ein Maß für den Siedepunkt.

0108962

392-93 - 31/82                           18. August 1983
CASCH/AGD/UMA

- 1 -

BATTELLE - INSTITUT E.V., Frankfurt/Main

Patentansprüche

1. Verfahren zur Überwachung von Bremsflüssigkeiten im Hinblick auf deren Siedepunkterniedrigung durch Wassseraufnahme, dadurch gekennzeichnet, daß in die Bremsflüssigkeit eine elektrisch betriebene Heizstrecke mit vergleichsweise geringen Abmessungen eingebracht und die die Heizstrecke unmittelbar umgebende Flüssigkeitsmenge bis zum örtlichen Sieden erhitzt wird, wobei die Temperatur der Heizstrecke durch das örtliche Sieden auf einen konstanten Wert festgehalten wird und daß der elektrische Widerstand der Heizstrecke bei dieser konstanten Temperatur als ein Maß für den Siedepunkt der Bremsflüssigkeit benutzt und die Unterschreitung eines Sollwertes des Siedepunkts angezeigt wird.

2. Verfahren nach Anspruch 1, dadurch gekennzeichnet, daß die Heizstrecke durch einen eingeprägten, konstanten Strom gespeist und der sich einstellende, dem Widerstand der Heizstrecke direkt proportionale Spannungsabfall als ein Maß für den Siedepunkt benutzt wird.

0108962

- 2 -

3. Verfahren nach Anspruch 1, <u>dadurch gekennzeichnet</u>, daß die Heizstrecke durch einen kontinuierlich, ansteigenden Strom gespeist wird und daß bei der Stabilisierung des Widerstandes der Heizstrecke die zugehörigen Werte für Strom und Spannungsabfall als Maß für den Siedepunkt benutzt werden.

4. Verfahren nach einem der Ansprüche 1 bis 3, <u>dadurch gekennzeichnet</u>, daß aus einer beginnenden Widerstandsfluktuation der Heizstrecke auf den Beginn des örtlichen Siedens geschlossen und der Meßvorgang ausgelöst wird.

5. Anordnung zur Durchführung des Verfahrens nach Anspruch 1 bis 4, <u>dadurch gekennzeichnet</u>, daß innerhalb eines die zu überwachende Bremsflüssigkeit enthaltenden Behälters (2) eine direkt oder indirekt elektrisch betriebene und von der Bremsflüssigkeit allseitig umgebene Heizstrecke (1) mit vergleichsweise geringen Abmessungen vorgesehen ist, deren elektrische Anschlüsse (4) druckdicht und elektrisch isoliert durch die Behälterwandung geführt sind und daß eine Elektronik (5) zur Stromversorgung und zur Bestimmung des Widerstandes der Heizstrecke sowie eine Meßwertanzeige vorhanden sind.

6. Anordnung nach Anspruch 5, <u>dadurch gekennzeichnet</u>, daß bei Kraftfahrzeugen in jedem Radbremszylinder (2) mindestens eine Heizstrecke (1) vorgesehen ist.

7. Anordnung nach Anspruch 5 oder 6, <u>dadurch gekennzeichnet</u>, daß die Heizstrecke (1) in Form eines Wendels ausgebildet ist.

8. Anordnung nach einem der Ansprüche 5 bis 7, <u>dadurch gekennzeichnet</u>, daß die Heizstrecke aus einem Metall mit hohen Temperaturkoeffizienten des elektrischen Widerstands, vorzugsweise aus Nickel, oder aus einem Kalt- oder Heißleitermaterial, das im Bereich des Sollwertes des Siedepunkts eine steile Kennlinie aufweist, besteht.

Fig.1